Europäisches Patentamt

European Patent Office

Office européen des brevets

(19)

(11) Numéro de publication : **0 409 054 B1**

(12) **FASCICULE DE BREVET EUROPEEN**

(45) Date de publication du fascicule du brevet :
**29.04.92 Bulletin 92/18**

(51) Int. Cl.$^5$ : **G01B 17/02, A61B 8/00,
G01N 29/00**

(21) Numéro de dépôt : **90113192.0**

(22) Date de dépôt : **11.07.90**

(54) **Procédé de traitement d'un signal électrique de distance.**

(30) Priorité : **20.07.89 FR 8909926**

(43) Date de publication de la demande :
**23.01.91 Bulletin 91/04**

(45) Mention de la délivrance du brevet :
**29.04.92 Bulletin 92/18**

(84) Etats contractants désignés :
**CH DE DK ES GB IT LI NL SE**

(56) Documents cités :
**EP-A- 0 150 997
FR-A- 2 308 909
US-A- 4 063 549**

(73) Titulaire : **ASULAB S.A.
Faubourg du Lac 6
CH-2501 Bienne (CH)**

(72) Inventeur : **Delon-Martin, Chantal
7, rue Arago
F-38000 Grenoble (FR)**
Inventeur : **Arditi, Marcel
Rue Lamartine 10
CH-1203 Genève (CH)**
Inventeur : **Farine, Pierre-André
Port-Roulant 12A
CH-2003 Neuchâtel (CH)**
Inventeur : **Meister, Jean-Jacques
Chemin de l'Eglise 10
CH-1066 Epalinges (CH)**
Inventeur : **Tardy, Yanik
Rue du Crêt 4bis
CH-1006 Lausanne (CH)**

(74) Mandataire : **de Raemy, Jacques et al
ICB Ingénieurs Conseils en Brevets SA
Passage Max. Meuron 6
CH-2001 Neuchâtel (CH)**

## Description

La présente invention est relative à un procédé pour mesurer la distance e séparant deux faces d'un objet, procédé comportant l'émission d'une onde ultrasonore se propageant à la vitesse cen direction de l'objet et la réception par un capteur des échos engendrés par réflexion de ladite onde sur les faces dudit objet, ledit capteur délivrant un signal électrique composite g(t) présentant une amplitude variant en fonction du temps.

Plusieurs méthodes ont déjà été proposées qui utilisent la voie ultrasonore pour mesurer la distance séparant deux faces d'un objet. Si la distance séparant les deux faces est importante, il n'y a en général pas de difficulté à interpréter le double signal reçu sous forme d'écho, car les faces engendrent deux pics nettement séparés dans le temps qui permettent, en connaissant la vitesse de propagation de l'onde dans les milieux où elle va se propager, de calculer la distance qui sépare les faces en question. Cependant, dès l'instant où lesdites faces sont très proches l'une de l'autre et espacées d'une distance de l'ordre de la longueur d'onde l'une de l'autre, les échos qu'elles produisent peuvent apparaître confondus.

Des épaisseurs de revêtement reposant sur un substrat, épaisseurs comprises entre 5 et 20 μm, ont pu être mesurées par interférométrie ultrasonore en utilisant une fréquence variant de 90 à 150 MHz. Une telle méthode est décrite dans l'article intitulé "Measurement of the thickness of thin layers by ultrasonic interferometry" paru dans J. Appl. Phys. 55 (1), January 1, 1984, pages 194-198. Cependant cette méthode ne fournit un résultat satisfaisant que si elle est appliquée à un objet stable dont les dimensions ne sont pas sujettes à variations pendant la mesure.

L'article intitulé "Digital signal processing method for multi-layered media thickness measurement" paru dans IEEE Ultrasonic Symposium 1988, pages 1027-1029, décrit une mesure ultrasonore opérée sur des couches minces de peinture appliquées dans l'industrie automobile. Dans ce cas les couches présentent des épaisseurs situées entre 20 et 110 μm. La fréquence centrale est ici de 75 MHz avec une largeur de bande de 100 MHz. La méthode de mesure est basée sur l'analyse d'un cepstre de puissance, défini comme étant la transformée de Fourier inverse du logarithme de la densité spectrale de puissance du signal radio fréquence. On est cependant ici en présence de couches superficielles qui permettent de travailler à haute fréquence, d'où un résultat permettant une bonne résolution. Cette méthode ne conviendrait pas à la mesure de l'épaisseur d'un objet situé à l'intérieur d'un milieu atténuant l'onde ultrasonore et qui obligerait ainsi à travailler à des fréquences plus basses (10 MHz) comme cela sera le cas dans une utilisation possible de la présente invention.

La méthode du cepstre fait également l'objet d'une thèse présentée le 9 février 1979 à l'Institut National Polytechnique de Grenoble par J.-C. Balluet, thèse intitulé "Les opérateurs cepstres, applications à la séparation d'échos rapprochés". Dans cette publication, on remarquera cependant que la méthode utilisée pour des échos très rapprochés donnent des résultats difficiles à interpréter et que les pics observés se trouvent à la limite de la visibilité.

Une méthode d'échographie ultrasonore appliquée in vivo sur des tissus vivants est décrite dans l'article intitulé "Range resolution improvement by a fast deconvolution method" paru dans Ultrasonic Imaging 6, 1984, pages 435-451. De nombreux problèmes de la physique mathématique peuvent être formulés au moyen d'une équation de convolution de la forme $y = h * x$, où x et y dénotent un système d'entrée et de sortie respectivement, et où h est la réponse impulsionnelle du système. La déconvolution est le problème inverse qui consiste à reconstruire l'entrée à partir d'une sortie mesurée expérimentalement. Grâce à la méthode exposée dans cet article, il a été possible d'étudier avec précison le fond de l'oeil et de suivre les variations de l'épaisseur d'une paroi artérielle durant le cycle cardiaque. La méthode proposée présente cependant le désavantage de séparer très difficilement des échos rapprochés dans le temps.

Des capteurs non invasifs permettant la mesure du diamètre intérieur d'une artère sont connus du brevet US-A-4 370 985. La méthode indiquée n'indique aucune solution pour mesurer l'épaisseur proprement dite de l'artère.

Du contenu de la littérature brièvement exposée ci-dessus, il ressort que les méthodes employées soit sont mal adaptées au milieu dans lequel doit être effectuée la mesure, soit donnent des résultats difficiles à interpréter quand il s'agit de mesure de très faibles épaisseurs. Pour obvier à ces inconvénients, la présente invention est caractérisée par le fait que le signal électrique composite reçu par le capteur est soumis à un traitement qui comporte la succession des étapes suivantes :

a) on transforme le signal électrique composite g(t) issu du capteur en un spectre fréquentiel G(f) dont l'amplitude est fonction de la fréquence, ledit spectre présentant une partie réelle Re[G(f)] et une partie imaginaire Im[G(f)],

b) on calcule le module d'ordre n du spectre fréquentiel $|G(f)|^n = \{Re[G(f)]^2 + Im[G(f)]^2\}^{n/2}$,

c) on calcule la dérivée logarithmique $(|G(f)|^n)'/|G(f)|^n$ du module calculé à l'étape b) pour séparer du signal électrique composite les échos proprement dits, ladite dérivée logarithmique présentant une partie utile sensiblement centrée sur la fréquence de l'onde ultrasonore et une partie bruitée située de part et d'autre de la partie utile, ladite partie utile comprenant un signal périodique auquel se superpose un signal sensiblement linéaire,

d) on applique à la dérivée logarithmique une fenêtre fréquentielle pour isoler sa partie utile et éliminer sa partie bruitée,

e) on applique au moins au signal périodique de ladite partie une transformation inverse à celle de l'étape a),

f) on détermine l'enveloppe de la transformation inverse, ladite enveloppe présentant une série de pics d'amplitudes variables espacés dans le temps,

g) on détermine l'écart temporel Δt séparant l'origine des temps du pic présentant la plus grande amplitude dû à la contribution du signal périodique de la partie utile, la distance e recherchée séparant les deux faces de l'objet étant exprimée par la relation e = c·Δt/2.

L'invention va être comprise maintenant à la lecture de la description qui suit, donnée à titre d'exemple et illustrée par le dessin dans lequel :

– la figure 1 est une vue schématique d'un exemple de dispositif de mesure montrant un objet dont il s'agit de mesurer l'épaisseur de la paroi, et un capteur délivrant un signal électrique composite à un ordinateur, ce signal subissant le traitement qui est l'objet de la présente invention.

– la figure 2 est un organigramme faisant état des diverses étapes du procédé selon l'invention et selon un exemple de traitement du signal,

– la figure 3 montre le signal composite délivré par le capteur quand l'épaisseur de la paroi est de 0,75 mm, la paroi étant ici celle d'un tube en matière plastique,

– la figure 4 montre le même signal pour une épaisseur de 0,3 mm,

– la figure 5 montre le même signal pour une épaisseur de 0,15 mm,

– la figure 6 est un diagramme montrant la partie réelle du spectre fréquentiel obtenu en transformant le signal issu du capteur pour le signal de la figure 5,

– la figure 7 montre la partie imaginaire du spectre fréquentiel obtenu en transformant le signal issu du capteur pour le signal de la figure 5,

– la figure 8 est un diagramme montrant le module carré du spectre fréquentiel montré aux figures 6 et 7,

– la figure 9 est un diagramme montrant la dérivée logarithmique du module carré montré à la figure 8,

– la figure 10 est un diagramme semblable à celui de la figure 9, dans lequel on a procédé à un redressement de la courbe,

– la figure 11 est un diagramme montrant le signal de la figure 10 après lui avoir appliqué une fenêtre fréquentielle,

– la figure 12 est une représentation de la transformation inverse du signal de la figure 11,

– la figure 13 est un diagramme où l'on a représenté l'enveloppe du signal de la figure 12 avec une échelle des abscisses dilatée, enveloppe permettant de calculer l'épaisseur de la paroi du tube,

– la figure 14 montre le diagramme enveloppe résultant d'une mesure semblable appliquée in vitro sur une artère d'animal, et

– la figure 15 est un diagramme obtenu dans les mêmes conditions que celles de la figure 14, mais pour lequel l'étape montrée en figure 10 n'a pas été effectuée.

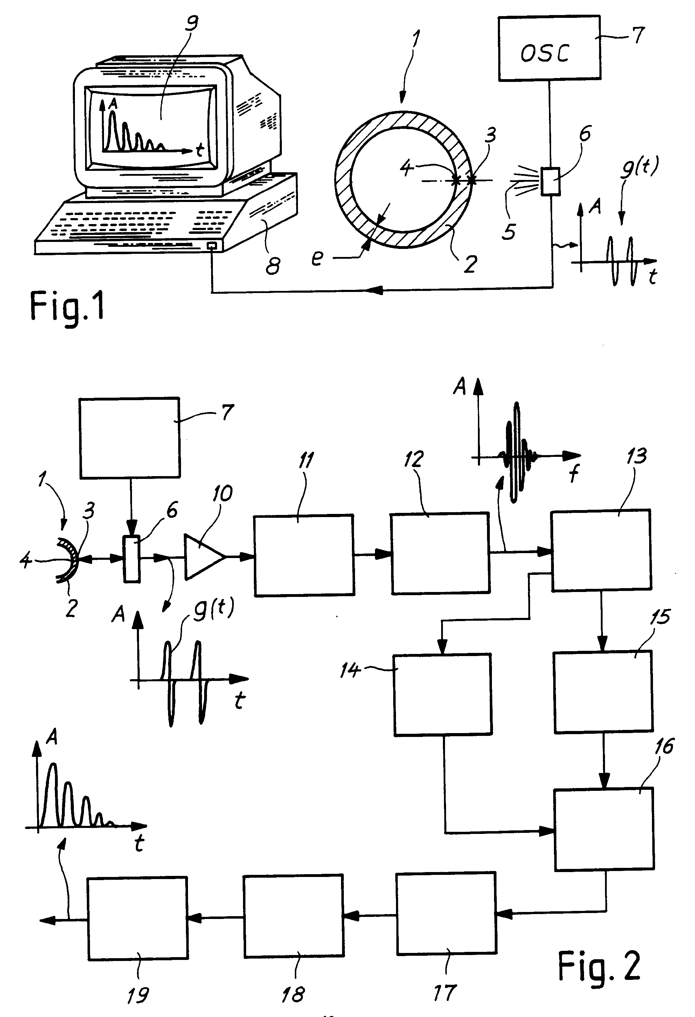

La figure 1 montre en coupe un objet 1 dont il s'agit de mesurer l'épaisseur de la paroi 2. La paroi est délimitée par une face externe 3 et par une face interne 4. Pour procéder à cette mesure on émet une onde ultrasonore 5 au moyen d'un capteur ultrasonore émetteur-récepteur 6, onde qui se propage à la vitesse c dans la direction de l'objet. En émission le capteur est excité par un oscillateur 7 dont la fréquence est de l'ordre de 10 MHz. Dans l'exemple, le capteur 6 sert également de récepteur des échos engendrés par réflexion de l'onde sur les faces 3 et 4 de l'objet. Ce capteur délivre un signal électrique composite g(t) qui présente une amplitude A variant en fonction du temps t. Ce signal électrique est soumis à un traitement selon un procédé qui est l'objet de la présente invention. Tout le traitement peut être fait au moyen d'un ordinateur personnel (PC) représenté en 8 sur la figure 1. A chaque étape du procédé il est possible, au moyen de l'affichage 9, de visualiser le calcul effectué . On mentionnera ici que tout le procédé selon l'invention peut être conduit par un ordinateur personnel IBM AT auquel on adjoint un coprocesseur mathématique du type 8087. D'autres appareils pourraient être utilisés, ceux qui viennent d'être indiqués ne constituant qu'un exemple possible.

L'organigramme de la figure 2 présente différents blocs qui illustrent chacun une opération du procédé. La disposition représentée est celle qui est la plus complète. La description qui va suivre indiquera cependant que certains blocs peuvent être supprimés sans s'écarter pour autant de l'idée principale de l'invention.

L'oscillateur 7 excite un capteur piézoélectrique 6 qui émet une onde ultrasonore de fréquence centrale d'environ 10 MHz. Ce même capteur recueille le signal g(t) rétrodiffusé par les faces 3 et 4 de la paroi 2 de l'objet 1. Ce signal présente une amplitude A variant en fonction du temps t et se présente sous forme analogique à la sortie du capteur 6. Après amplification dans l'amplificateur 10, il est commode, mais pas absolument obligatoire, de numériser le signal g(t) au moyen d'un convertisseur analogique - numérique 11. Dans le cas pris ici en exemple, le signal est échantillonné à 100 MHz sur 8 bits, puis stocké dans une mémoire comportant 2048 points.

L'objet utilisé ici pour expliquer le procédé de l'invention est un tube en matière plastique dont on se propose de mesurer l'épaisseur de la paroi. Comme on peut mesurer cette épaisseur par d'autres moyens classiques, comme par exemple au moyen d'un micromètre, on pourra confronter les résultats de mesure des deux systèmes, ce qui permettra de vérifier les résultats obtenus selon le procédé de l'invention.

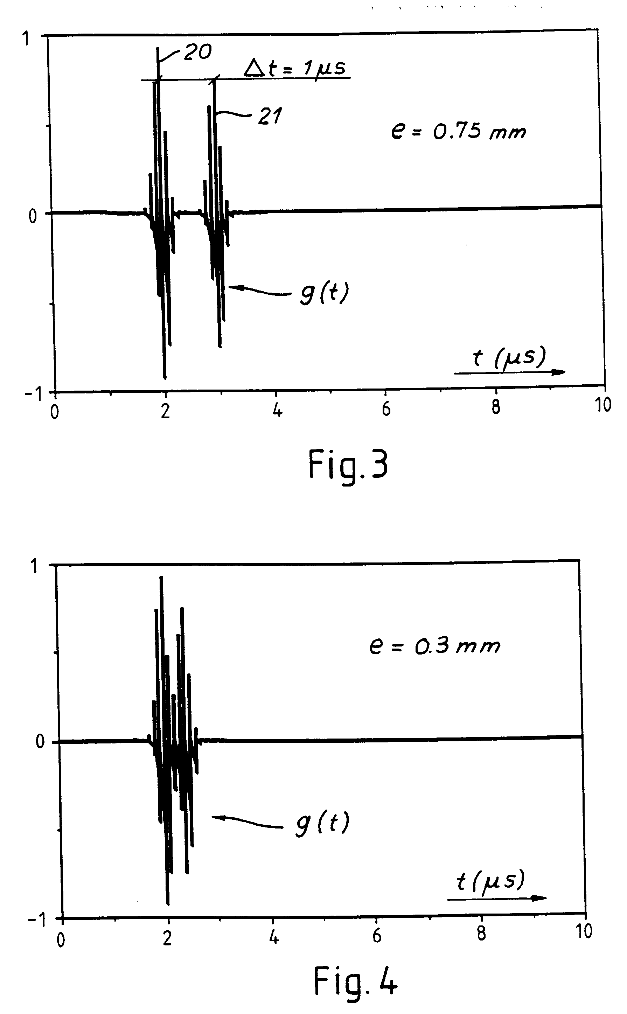

Les diagrammes présentés aux figures 3, 4 et 5 montrent chacun le signal g(t) après numérisation et pour trois cas particuliers de l'épaisseur de la paroi du tube. Dans ces diagrammes on a indiqué l'amplitude du signal en ordonnée (valeur relative) et le temps en abscisse (valeur en microsecondes).

La figure 3 montre le signal g(t) pour une épaisseur de paroi e = 0,75 mm. On distingue ici nettement les deux échos 20 et 21 renvoyés par les faces extérieure 3 et intérieure 4 de la paroi. Si l'on mesure sur le diagramme le temps $\Delta t$ séparant les deux pics principaux du signal, on trouve $\Delta t = 1\mu s$. Comme on connaît par ailleurs la vitesse c de propagation de l'onde, qui est égale, dans le cas particulier pris en exemple, à 1,5 mm/$\mu$s, on calcule immédiatement l'épaisseur de la paroi qui est égale à e = c·$\Delta t$/2, la division par 2 prenant en compte l'aller et le retour de l'onde. En remplaçant dans la formule cet $\Delta t$ par 1,5 et 1 respectivement, on trouve pour l'épaisseur e la valeur de 0,75 mm. On constate que dans le cas où les échos sont nettement départagés la mesure de l'épaisseur est immédiate et qu'il n'est pas nécessaire de recourir au procédé selon l'invention.

La figure 4 montre le signal g(t) pour une épaisseur de paroi de 0,3 mm. Ici les deux échos sont plus rapprochés et si l'on se livre à la même opération qui précédemment, on pourra obtenir un résultat correspondant plus ou moins à la réalité selon les pics que l'on aura choisis.

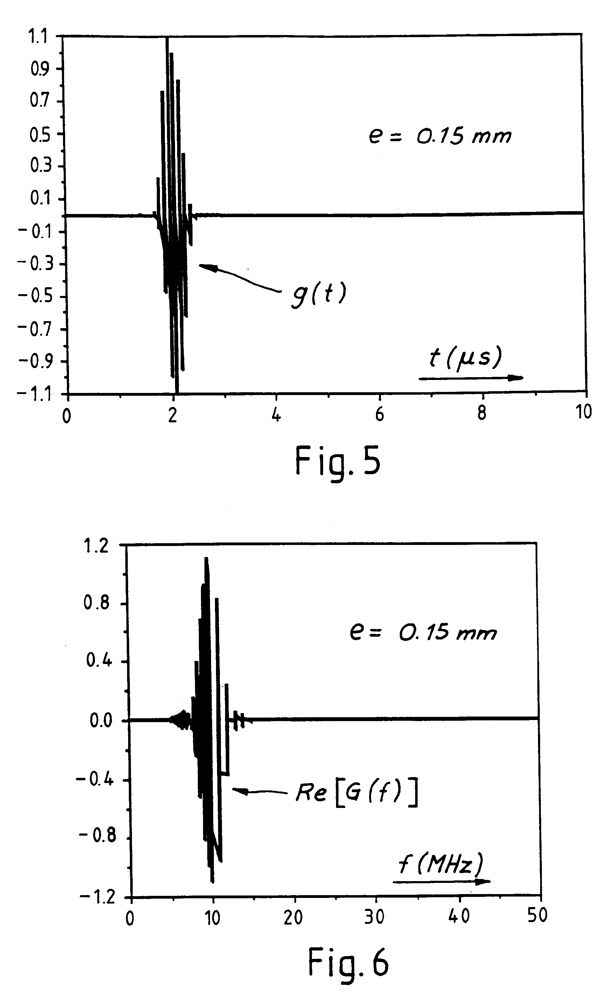

La figure 5 montre le signal g(t) pour une épaisseur de paroi de 0,15 mm. Ici les deux pics à choisir sont difficiles, voire impossibles à déterminer. Les échos apparaissent comme confondus et c'est le but de la présente invention de proposer une méthode de calcul capable d'isoler les pics à prendre en considération pour mesurer épaisseur de la paroi. Cette méthode va être appliquée maintenant au cas du tube donnant comme signal initial g(t) celui représenté à la figure 5. Pour cela, on se reportera à la figure 2 ainsi qu'à la série de diagrammes montrés aux figures 5 à 13.

## Exposé de la méthode

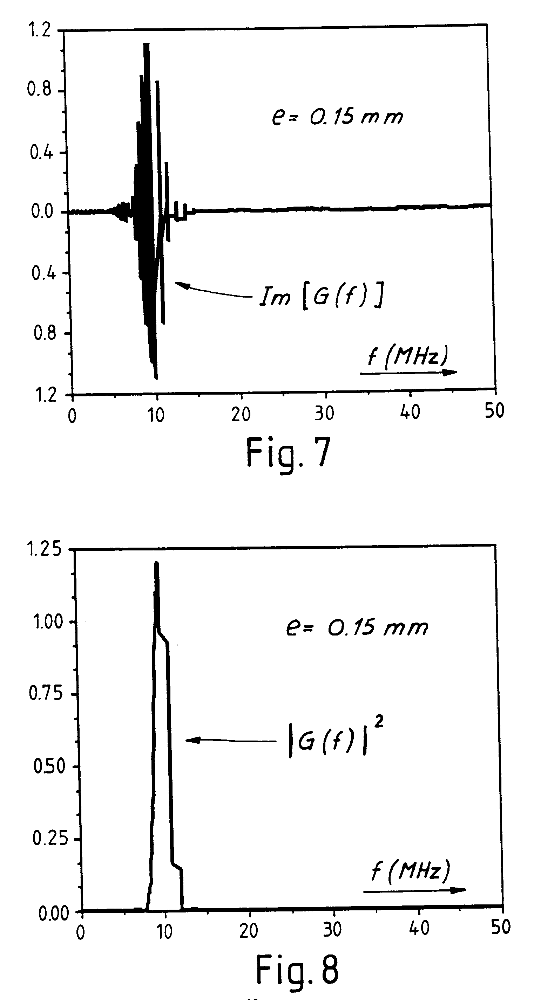

a) Le signal électrique composite g(t) présente à la sortie du bloc 11 et représenté à la figure 5 subit d'abord une opération dans le bloc 12, cette opération consistant à transformer ledit signal dépendant du temps en un spectre fréquentiel dont l'amplitude A dépend de la fréquence f. Cette opération est généralement précédée d'une correction d'offset, dans le cas où le signal g(t) en est pourvu. Tout à fait généralement, il peut s'agir ici d'une transformation en z introduit par Hurewiez pour l'étude des servomécanismes. A ce sujet, on se reportera à l'ouvrage intitulé "Theory of servomechanisms" édité par McGraw-Hill Book Co., Inc. New York, ch.5 ; 1947. Dans le cas qui intéresse ici, on utilise avec profit un cas particulier de la transformation en z qui est la bien connue transformation de Fourier. Après transformation, et à la sortie du bloc 12, le signal G(f) se présente sous la forme d'une partie réelle Re[g(f)] représentée à la figure 6 et d'une partie imaginaire Im[G(f)] représentée à la figure 7.

b) Les deux parties réelle et imaginaire du spectre fréquentiel G(f) sont ensuite introduites dans un bloc 13, (figure 2) qui a pour but de calculer le module d'ordre n dudit spectre selon la relation $|G(f)|^n = \{Re[G(f)]^2 + Im[G(f)]^2\}^{n/2}$. Dans le cas particulier, on utilisera avantageusement le module carré du spectre fréquentiel en faisant n = 2. Ce module carré est représenté à la figure 8 qui montre un pic très prononcé sur la fréquence centrale d'émission du capteur ultrasonore (environ 10 MHz).

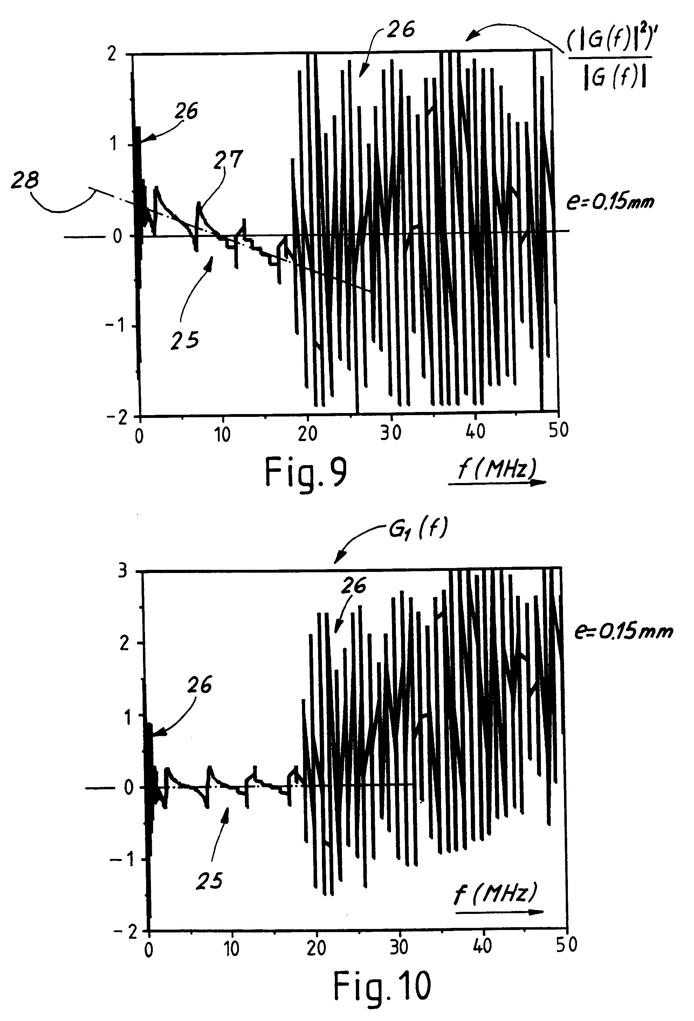

c) Le module obtenu à la sortie du bloc 13 est ensuite introduit dans un bloc 15 (figure 2) dont le rôle est le calcul de la dérivée logarithmique du module, définie par le quotient de la dérivée du module $(|G(f)|^n)'$ sur le module $|G(f)|^n$ proprement dit. Il s'agit là de l'étape la plus importante de l'invention grâce à laquelle il va être possible de séparer du signal électrique composite les deux échos recherchés. A la sortie du bloc 15 la dérivée logarithmique se présente sous la forme représentée à la figure 9 qui montre distinctement deux parties : une partie utile 25, sensiblement centrée sur la fréquence de l'onde ultrasonore et une partie bruitée 26, située de part et d'autre de la partie utile. La partie utile 25, qui est la partie intéressante, comprend un signal périodique 27 auquel se superpose un signal sensiblement linéaire 28. Ces deux composantes de la partie utile s'expliquent physiquement : le signal de départ g(t) comporte en effet un double pic à détecter, ce double pic se trouvant mélangé au signal émis par le capteur qui peut être assimilé à un sinus modulé en amplitude par une gaussienne. La transformation de Fourier G(f) du signal g(t) présente encore un mélange et lorsqu'on en calcule la dérivée logarithmique, les pics se transforment en un signal périodique 27 et le signal émis par le capteur en un signal sensiblement linéaire 28. On notera ici que cette séparation est aisément réalisable dans le domaine fréquentiel, alors qu'elle n'est pas possible dans le domaine temporel, ce qui explique la transformation opérée à l'étape a).

Dans une version préférée mais non obligatoire de l'invention, on supprime alors le signal linéaire 28 qui se présente sur le diagramme 9 sous une forme de pente. La suppression de cette pente présente un avantage qui apparaîtra par la suite. Plusieurs méthodes peuvent être mises en oeuvre pour supprimer cette pente ou signal linéaire, ces méthodes aboutissant au signal représenté à la figure et référencé $G_1(f)$.

Une première méthode consiste à visualiser sur un écran le signal représenté en figure 9, puis de procéder à la suppression du signal linéaire en redressant ce signal au moyen d'essais ou d'approximations successifs jusqu'à ce qu'on obtienne un résultat graphiquement satisfaisant du type de celui montré à la figure 10. Cela sera le cas quand la pente 28 sera confondue avec l'axe des fréquences. Cette opération est symbolisée par le bloc 16 de la figure 2.

Une deuxième méthode utilise une voie mathématique. On commence par calculer, à partir de l'expression du module carré ( $n = 2$ ) obtenu après l'étape b), le moment m1 d'ordre 1 et le moment m2 d'ordre 2 dudit module. Ces moments sont donnés par la relation suivante en faisant $i = 1$ pour m1 et $i = 2$ pour m2 :

$$m_n = \frac{\displaystyle\sum_{i=-\infty}^{i=+\infty} |G(f_i)|^2 \cdot f_i^n}{\displaystyle\sum_{i=-\infty}^{i=+\infty} |G(f_i)|^2}$$

On détermine ensuite la droite passant par $f = m1$ et ayant pour pente $p = k \cdot m2$, droite qui s'exprime par la relation $p(f-m1)$, où $f$ est égal à la fréquence et $k$ est une constante. On soustrait enfin la droite définie ci-dessus de la dérivée logarithmique obtenue à l'étape c) de façon à avoir :

$$G_1(f) = (|G(f)|^2)'/|G(f)|^2 - p(f - m1)$$

Dans l'organigramme de la figure 2, le bloc 14 représente le calcul des moments m1 et m2 du module obtenu au bloc 13 et le bloc 16 représente la suppression proprement dite de la pente ou signal linéaire, qui s'opère par la soustraction indiquée ci-dessus.

Une troisième méthode utilise les caractéristiques de la partie sensiblement linéaire connues à priori par une mesure de référence.

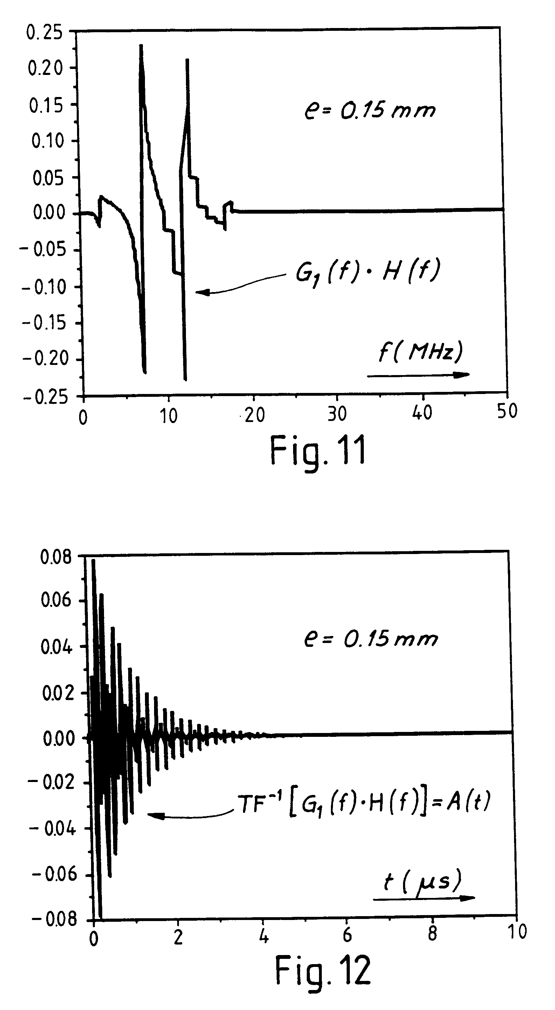

d) A la dérivée logarithmique obtenue à l'étape c), on applique une fenêtre fréquentielle pour isoler sa partie utile 25 et éliminer sa partie bruitée 26. Cette opération a lieu immédiatement après l'étape c) représentée par le bloc 15 dans le cas où on ne procède pas à la correction de pente évoquée plus haut. Dans le cas où la correction de pente est effectuée (bloc 16), la fenêtre fréquentielle peut être appliquée indifféremment avant ou après cette correction. Dans l'organigramme de la figure 2 qui prévoit une correction de pente, on a opté pour l'application de la fenêtre (bloc 17 de la figure 2) après ladite correction (bloc 16) et le diagramme de la figure 11 montre le signal après l'application de la fenêtre. Cette fenêtre est un filtre passe-bande qui, comme le montre la figure 11 a fait disparaître la partie bruitée qui se trouvait de part et d'autre de la partie utile. Avantageusement, la fenêtre présente une forme en cloche du type de la fenêtre dite de Hanning (voir l'ouvrage intitulé : "The measurement of power spectra" de R. B. Blackman et J. W. Tuckey, Dover Publications New York 1958). D'autres fenêtres sont utilisables et l'on consultera pour cela l'ouvrage de M. Kunt intitulée "Traitement numérique des signaux" édité par Dunod, 1981.

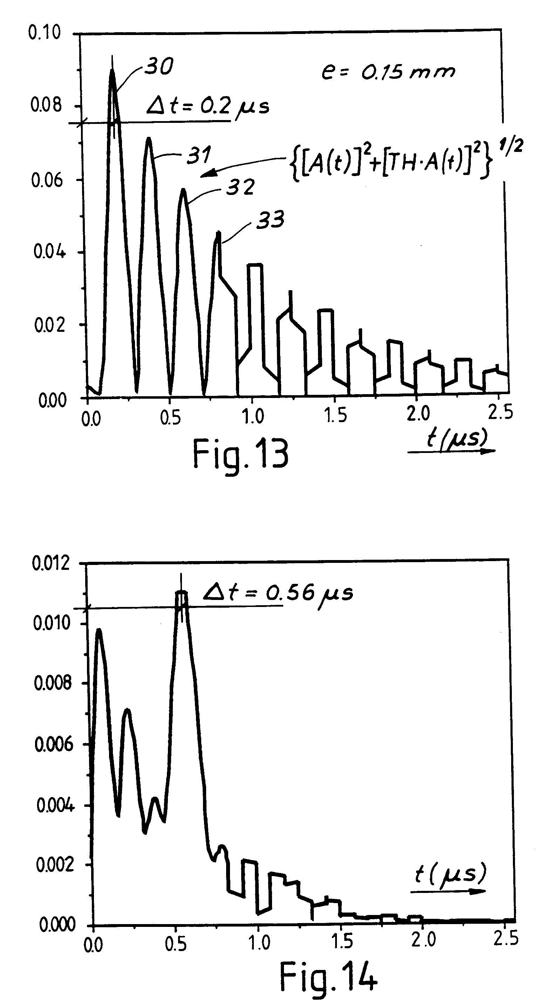

e) Le signal après fenêtre est rendu exploitable si l'on revient maintenant dans le domaine temporel, ce que se charge de faire le bloc 18 de la figure 2 qui applique au signal une transformation inverse à celle qui a été décrite à l'étape a). Par analogie à ce qui a été dit plus haut cette opération peut être effectuée très généralement par une transformation inverse en z ou, comme dans le cas pris ici en exemple, par une transformation de Fourier inverse. Le signal après transformation est montré à la figure 12. Si l'on désigne par $TF^{-1}$ la transformation de Fourier inverse, par $G_1(f)$ la dérivée logarithmique du module carré après correction de pente et par $H(f)$ la fenêtre fréquentielle, le signal de la figure 12 peut être exprimé par la relation

$$A(t) = TF^{-1}[G_1(f) \cdot H(f)]$$

f) L'étape suivante du procédé consiste à déterminer l'enveloppe de la transformation inverse opérée à l'étape e). Cette opération est effectuée par le bloc 19 de la figure 2 et se présente comme montré à la figure 13. Comme le montre cette dernière figure, l'enveloppe présente une série de pics 30, 31, 32, etc.., d'amplitudes variables et espacés dans le temps. Pour y parvenir on se servira avantageusement de la transformation dite de Hilbert décrite dans l'ouvrage "Probability, random variables and stochastic process"de A. Papoulis édité par McGraw-Hill, 1984. Si l'on désigne par TH la transformation de Hilbert, l'enveloppe de la figure 13 s'exprime par :

$$\{[A(t)]^2 + [TH \cdot A(t)]^2\}^{1/2}$$

la valeur de $A(t)$ étant celle obtenue ci-dessus.

g) A partir de l'enveloppe obtenue à l'étape précédente, on détermine l'écart temporel $\Delta t$ séparant l'origine des temps du pic 30 présentant la plus grande amplitude, pic dû à la contribution du signal périodique de la partie utile définie plus haut, en rappelant que c'est dans le signal périodique de la partie utile que se trouvent les échos recherchés. L'origine des temps est ici une origine relative qui correspond au premier écho. L'intervalle $\Delta t$ mesuré correspond alors bien à l'intervalle séparant les deux échos. Cette origine diffère de celle représentée à la figure 5. Cela provient du fait qu'en utilisant le module carré calculé à l'étape b) on perd l'information qui contient le temps séparant l'origine des temps montrée à la figure 5 du premier écho.

Dans le cas particulier, puisque la pente du signal utile a été supprimée, le pic de plus grande amplitude dû à la contribution du signal périodique est bien le premier rencontré et référencé 30, la distance e entre les deux faces 3 et 4 de la paroi 2 du tube 1 étant exprimée par la relation $e = c \cdot \Delta t/2$. Sur le graphique de la figure 13 on mesure $\Delta t = 0{,}2$ $\mu s$. Si $c = 1{,}5$ mm/$\mu s$ comme indiqué plus haut, on trouve $e = 1{,}5 \cdot 0{,}2/2 = 0{,}15$ mm ce qui correspond bien à l'épaisseur du tube dont on savait par d'autres moyens de mesure, qu'elle était égale à 0,15 mm.

Considérations annexes

Le tube en plastique utilisé plus haut pour expliquer la méthode de l'invention donne des signaux bien définis dus particulièrement à la rigidité du tube soumis à la mesure. Dans de nombreux cas, l'objet dont il s'agit de mesurer l'épaisseur est dépourvu de rigidité ; de plus, il est généralement inaccessible et ne permet pas une mesure par des moyens de mesure mécaniques. C'est le cas par exemple d'une artère in vivo dont il s'agit de mesurer de manière non invasive l'épaisseur de la paroi.

La figure 14 présente le signal enveloppe obtenu à l'avant-dernière étape du procédé selon l'invention sur une artère fémorale de porc in vitro. Dans ce cas, il a été effectué une correction de pente comme indiqué plus haut à l'étape c). L'enveloppe montre plusieurs pics et sans conteste, c'est le pic de plus grande amplitude noté à 0,56 $\mu s$ qui doit être pris en considération.

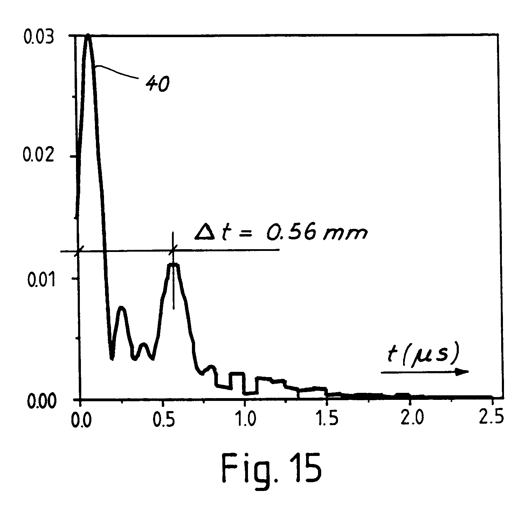

Le diagramme de la figure 15 est une enveloppe provenant de la même artère mais où la correction de pente n'a pas été effectuée. On retrouve bien à 0,56 $\mu s$ un pic correspondant à celui recherché, mais ce pic est précédé par un autre pic 40 de plus grande amplitude, pic provenant de la contribution de la partie linéaire (ou de la pente) du signal utile qui est prépondérante. Cet enseignement milite en faveur de l'utilisation systématique de la correction de pente dans tous les cas où l'objet à mesurer présente peu de stabilité et/ou n'est pas accessible.

On a déjà mentionné que le procédé peut être utilisé pour la mesure de l'épaisseur d'une artère. Le même procédé peut être mis à profit pour la mesure du diamètre intérieur de l'artère et, en général, pour la mesure de tout objet présentant une couche mince dont il s'agit de mesurer l'épaisseur.

**Revendications**

1. Procédé pour mesurer la distance e séparant deux faces d'un objet, procédé comportant l'émission d'une onde ultrasonore se propageant à la vitesse c en direction de l'objet et la réception par un capteur des échos engendrés par réflexion de ladite onde sur les faces dudit objet, ledit capteur délivrant un signal électrique composite g(t) présentant une amplitude variant en fonction du temps, caractérisé par le fait que ledit signal électrique composite est soumis à un traitement comportant la succession des étapes suivantes :

a) on transforme le signal électrique composite g(t) issu du capteur en un spectre fréquentiel G(f) dont l'amplitude est fonction de la fréquence, ledit spectre présentant une partie réelle Re[G(f)] et une partie imaginaire Im[G(f)],

b) on calcule le module d'ordre n du spectre fréquentiel $|G(f)|^n = \{Re\ [G(f)]^2 + Im[G(f)]^2\}^{n/2}$,

c) on calcule la dérivée logarithmique $(|G(f)>|^n)'/|G(f)|^n$ du module calculé à l'étape b) pour séparer du signal électrique composite les échos proprement dits, ladite dérivée logarithmique présentant une partie utile sensiblement centrée sur la fréquence de l'onde ultrasonore et une partie bruitée située de part et d'autre de la partie utile, ladite partie utile comprenant un signal périodique auquel se superpose un signal sensiblement linéaire,

d) on applique à la dérivée logarithmique une fenêtre fréquentielle pour isoler sa partie utile et éliminer sa partie bruitée,

e) on applique au moins au signal périodique de ladite partie utile une transformation inverse à celle de l'étape a),

f) on détermine l'enveloppe de la transformée inverse, ladite enveloppe présentant une série de pics d'amplitudes variables espacés dans le temps,

g) on détermine l'écart temporel $\Delta t$ séparant l'origine des temps du pic présentant la plus grande amplitude dû à la contribution du signal périodique de la partie utile, la distance e recherchée séparant les deux faces de l'objet étant exprimée par la relation $e = c \cdot \Delta t/2$.

2. Procédé selon la revendication 1, caractérisé par le fait qu'immédiatement avant ou après l'étape d), on supprime le signal sensiblement linéaire apparaissant à l'étape c), le pic de plus grande amplitude déterminé à l'étape g) étant alors le pic de plus grande amplitude de l'enveloppe obtenue à l'étape f).

3. Procédé selon la revendication 1 ou la revendication 2, caractérisé par le fait que l'étape a) est précédée par une numérisation du signal électrique composite, numérisation réalisée au moyen d'un convertisseur analogique-numérique et que le signal ainsi obtenu est stocké dans une mémoire.

4. Procédé selon la revendication 1 ou la revendication 2, caractérisé par le fait que la transformation apparaissant à l'étape a) est une transformation de Fourier et que la transformation inverse apparaissant à l'étape e) est une transformation de Fourier inverse.

5. Procédé selon la revendication 1 ou la revendication 2, caractérisé par le fait que l'enveloppe apparaissant à l'étape f) utilise une transformation de Hilbert du signal apparaissant à l'étape e).

6. Procédé selon la revendication 1 ou la revendication 2, caractérisé par le fait que la fenêtre fréquentielle apparaissant à l'étape d) est une fenêtre de Hanning.

7. Procédé selon la revendication 2, caractérisé par le fait qu'on visualise la partie utile du signal apparaissant à l'étape c) et qu'on procède à la suppression du signal sensiblement linéaire au moyen d'essais successifs jusqu'à l'obtention d'un résultat graphiquement satisfaisant.

8. Procédé selon la revendication 2, caractérisé par le fait que la suppression du signal sensiblement linéaire apparaissant après l'étape c) ou l'étape d) est obtenue par le succession des étapes suivantes :

h) de l'expression du module d'ordre n obtenu à l'étape b) pour lequel on pose n = 2, on calcule le moment m1 d'ordre 1 et le moment m2 d'ordre 2,

i) on détermine la droite passant par f = m1 ayant pour pente p = k·m2, droite qui s'exprime par la relation p(f - m1), où f est égal à la fréquence et k est une constante, et

j) on soustrait de la dérivée logarithmique obtenue à l'étape c) la droite obtenue à l'étape i).

9. Procédé selon la revendication 2, caractérisé par le fait qu'on procède à la suppression du signal sensiblement linéaire en utilisant les caractéristiques dudit signal connues à priori par une mesure de référence.

10. Utilisation du procédé selon l'une quelconque des revendications 1 à 9 pour la mesure non invasive de l'épaisseur de la paroi d'une artère.

11. Utilisation du procédé selon l'une quelconque des revendications 1 à 9 pour la mesure non invasive du diamètre intérieur d'une artère.

12. Utilisation du procédé selon l'une quelconque des revendications 1 à 9 pour la mesure de l'épaisseur de couches minces.

**Patentansprüche**

1. Verfahren zum Messen der Distanz e zwischen zwei Seiten eines Gegenstandes, welches Verfahren die Aussendung einer Ultraschallwelle umfaßt, die sich mit der Geschwindigkeit c in Richtung des Gegenstandes ausbreitet und den Empfang, mittels eines Aufnehmers, von Echos umfaßt, erzeugt durch Reflexion der Welle auf den Seiten des Gegenstandes, welcher Aufnehmer ein elektrisches zusammengesetztes Signal g(t) liefert mit einer zeitabhängig variablen Amplitude, **dadurch gekennzeichnet**, daß das zusammengesetzte elektrische Signal einer Verarbeitung unterworfen wird, welche die Abfolge der nachstehenden Schritte umfaßt:

a) Man transformiert das elektrische zusammengesetzte Signal g(t), abgegeben von dem Aufnehmer, in ein Frequenzspektrum G(f), dessen Amplitude eine Funktion der Frequenz ist, welches Spektrum einen reellen Anteil Re[G(f)] und einen imaginären Anteil Im[G(f)] aufweist,

b) man berechnet den Ordnungsmodul n des Frequenzspektrums $|G(f)|^n = \{Re[G(f)]^2 + Im[G(f)]^2\}^{n/2}$,

c) man berechnet die logarithmische Ableitung $(|G(f)|^n)'/|G(f)|^n$ des in Schritt b) berechneten Moduls zum Abtrennen der eigentlichen Echos von dem zusammengesetzten elektrischen Signal, welche logarithmische Ableitung einen brauchbaren Anteil umfaßt, der im wesentlichen auf die Frequenz der Ultraschallwelle zentriert ist und einen verrauschten Anteil aufweist, der sich beidseits des brauchbaren Anteils befindet, wobei der brauchbare Anteil ein periodisches Signal umfaßt, dem sich ein im wesentlichen lineares Signal überlagert,

d) man wendet auf die logarithmische Ableitung ein Frquenzfenster an, um den brauchbaren Anteil zu isolieren und den verrauschten Anteil zu eliminieren,

e) man wendet auf mindestens das periodische Signal des brauchbaren Anteils eine Transformation an invers zu der des Nachschritts a),

f) man bestimmt die Umhüllende der inversen Transformierten, welche Umhüllende eine Serie von Amplitudenspitzen aufweist, die zeitlich variable Abstände besitzen,

g) man bestimmt den zeitlichen Abstand Δt, der den Anfang der Zeiten der Spitze trennt, welche die größte Amplitude aufweist, infolge des Beitrags des periodischen Signals des brauchbaren Anteils, wobei die gesuchte Distanz e, welche die beiden Seiten des Gegenstandes voneinander trennt, ausgedrückt wird durch die Beziehung e = c · Δt/2.

2. Verfahren nach Anspruch 1, dadurch gekennzeichnet, daß unmittelbar vor oder nach dem Schritt d) man das im wesentlichen lineare in Schritt c) erscheinende Signal unterdrückt, wobei die Spitze mit der größten Amplitude, die in Schritt g) bestimmt wird, demgemäß die Spitze der größten Amplitude der in Schritt f) abgeleiteten Umhüllenden ist.

3. Verfahren nach Anspruch 1 oder Anspruch 2, dadurch gekennzeichnet, daß dem Schritt a) eine Numerisierung des zusammengesetzten elektrischen Signals vorausgeht, welche Numerisierung realisiert wird mittels eines analog numerischen Wandlers und daß das auf diese Weise erhaltene Signal in einem Speicher abgespeichert wird.

4. Verfahren nach Anspruch 1 oder Anspruch 2, dadurch gekennzeichnet, daß die in Schritt a) erscheinende Transformation eine Fourier Transformation ist und daß die inverse Transformation in Schritt e) eine inverse Furier Transformation ist.

5. Verfahren nach Anspruch 1 oder Anspruch 2, dadurch gekennzeichnet, daß die in Schritt f) erscheinende Umhüllende eine Hilbert Transformation des Signals verwendet, das in Schritt e) erscheint.

6. Verfahren nach Anspruch 1 oder Anspruch 2, dadurch gekennzeichnet, daß das in Schritt d) erscheinende Frequenzfenster ein Hanning Fenster ist.

7. Verfahren nach Anspruch 2, dadurch gekennzeichnet, daß man den brauchbaren Anteil des in Schritt c) erscheinenden Signal visualisiert und daß man die Unterdrückung des im wesentlichen linearen Signals mittels aufeinanderfolgender Versuche vornimmt bis zum Erhalten eines grafisch befriedigenden Resultats.

8. Verfahren nach Anspruch 2, dadurch gekennzeichnet, daß die Unterdrückung des im wesentlichen linearen Signals, das nach Schritt c) oder d) erscheint, durch eine Abfolge der nachstehenden Schritte erhalten wird:

h) Man berechnet aus dem Ausdruck des Ordnungsmoduls n, den man in Schritt b) erhält, für den man n = 2 setzt, das Moment m1 der Ordnung 1 und das Moment m2 der Ordnung 2,

i) man bestimmt die Gerade, die durch f = m1 verläuft mit einer Steigung p = k·m2, welche Gerade sich ausdrückt durch die Beziehung p(f - m1), worin f gleich der Frequenz und k eine Konstante sind, und

j) man subtrahiert von der in Schritt c) gewonnenen logarithmischen Ableitung die in Schritt i) erhaltene Gerade.

9. Verfahren nach Anspruch 2, dadurch gekennzeichnet, daß man die Unterdrückung des im wesentlichen linearen Signals unter Verwendung der Charakteristiken des von vornherein durch eine Referenzmessung bekannten Signals.

10. Verwendung des Verfahrens nach einem der vorangehenden Ansprüche 1 bis 9 für die nicht invasive Messung der Dicke der Wandung einer Arterie.

11. Verwendung des Verfahrens nach einem der Ansprüche 1 bis 9 für die nicht invasive Messung des Innendurchmessers einer Arterie.

12. Verwendung des Verfahrens nach einem der Ansprüche 1 bis 9 für die Messung der Dicke dünner Schichten.

**Claims**

1. Method for measuring the distance e separating two faces of an object which method includes the emission of an ultrasonic wave propagating at the velocity c toward the object and the reception by a sensor of the echoes generated by reflection of said wave from the faces of said object, said sensor delivering a composite electrical signal g(t) showing an amplitude varying as a function of time, characterized by the fact that said composite electrical signal is processed by the following sequence of steps :

a) transforming the composite electrical signal g(t) coming from the sensor into a frequency spectrum G(f) the amplitude of which is a function of the frequency, said spectrum exhibiting a real part Re[G(f)] and an imaginary part Im[G(f)],

b) calculating the order n modulus of the frequency spectrum $|G(f)|^n = \{Re\ [G(f)]^2 + Im[G(f)]^2\}^{n/2}$,

c) calculating the logarithmic derivative $(|G(f)|^n)'/|G(f)|^n$ of the modulus calculated in step b) in order to separate the existing echoes from the composite electrical signal, said logarithmic derivative exhibiting a useful part centered substantially on the frequency of the ultrasonic wave and a part having noise located on either side of the useful part, said useful part comprising a periodic signal onto which a substantially linear signal is superposed,

d) applying a frequency window to the logarithmic derivative in order to isolate the useful part thereof and to eliminate the part having noise,

e) applying a transformation inverse to that of step a) at least to the periodic signal of said useful part,

f) determining the envelope of the inverse transformation, said envelope exhibiting a series of variable amplitude peaks spaced out over time,

g) determining the temporal spread $\Delta t$ separating the time origin from the peak showing the greatest amplitude due to the contribution of the periodic signal from the useful part, the sought for distance e separating the two faces of the object being expressed by the relation e = c·$\Delta t$/2.

2. Method according to claim 1, characterized by the fact that immediately before or after step d) the substantially linear signal appearing at step c) is suppressed, the peak having the greatest amplitude as determined in step g) being then the greatest amplitude peak of the envelope obtained in step f).

3. Method according to claim 1 or to claim 2, characterized by the fact step a) is preceded by a digitalization of the composite electrical signal, said digitalization being brought about by means of an analog-digital converter and the signal thus obtained being stored in a memory.

4. Method according to claim 1 or to claim 2, characterized by the fact the transformation effected in step a) is a Fourier transformation and that the inverse transformation effected in step e) is an inverse Fourier transformation.

5. Method according to claim 1 or to claim 2, characterized by the fact the envelope appearing in step f) employs a Hilbert transformation of the signal appearing in step e).

6. Method according to claim 1 or to claim 2, characterized by the fact the frequency window appearing in step d) is a Hanning window.

7. Method according to claim 2, characterized by the fact the useful part of the signal appearing at step c) is visualized and that one proceeds to suppress the substantially linear signal by means of successive trials until a graphically satisfactory signal is obtained.

8. Method according to claim 2, characterized by the fact suppression of the substantially linear signal appearing after step c) or step d) is obtained by the following sequence of steps :

h) calculating from the expression of the order n modulus obtained in step b), for which one places $n = 2$, the order 1 moment $m1$ and the order 2 moment $m2$,

i) determining the line guided by $f = m1$ having as slope $p = k-m2$, said line being expressed by the relation $p(f - m1)$ where $f$ is equal to the frequency and $k$ is a constant, and

j) subtracting the line obtained in step i) from the logarithmic derivative obtained in step c).

9. Method according to claim 2, characterized by the fact the substantially linear signal is suppressed by using the characteristics of said signal known a priori by a reference measurement.

10. Using the method according to claim 1 up to claim 9 for the non-invasive measurement of the wall thickness of an artery.

11. Using the method according to claim 1 up to claim 9 for the non-invasive measurement of the interior diameter of an artery.

12. Using the method according to claim 1 up to claim 9 for the measurement of the thickness of thin layers.

Fig.1

Fig. 2

Fig.3

Fig. 4

$e = 0.15 \, mm$

$g(t)$

$t \, (\mu s)$

Fig. 5

$e = 0.15 \, mm$

$Re \, [G \, (f)]$

$f \, (MHz)$

Fig. 6

Fig. 7

Fig. 8

Fig. 9

Fig. 10

$e = 0.15 \, mm$

$G_1(f) \cdot H(f)$

$f(MHz)$

Fig. 11

$e = 0.15 \, mm$

$TF^{-1}[G_1(f) \cdot H(f)] = A(t)$

$t(\mu s)$

Fig. 12

Fig.13

Fig.14

16

$\Delta t = 0.56\, mm$

$t\,(\mu s)$

Fig. 15